# EUROPÄISCHE PATENTSCHRIFT

(11) **EP 1 115 352 B1**
(45) Veröffentlichungstag und Bekanntmachung des Hinweises auf die Patenterteilung: **06.12.2006**
(21) Anmeldenummer: 99929253.5
(22) Anmeldetag: 17.06.1999
(51) Int. Cl.: A61F 2/06

(54) **PROTHESEN-SCHLAUCHVERBINDUNG**
PROSTHETIC TUBE CONNECTION
RACCORDEMENT TUBULAIRE PROTHETIQUE

(30) Priorität: 21.09.1998 DE 29817771 U
(43) Veröffentlichungstag der Anmeldung: 18.07.2001
(73) Patentinhaber: Metz, Katharina, 15741 Bestensee (DE)
(72) Erfinder: GUSSMANN, Andreas, D-14532 Kleinmachnow (DE); METZ, Ludwig, D-15741 Bestensee (DE)
(74) Vertreter: Manitz, Finsterwald & Partner GbR
(86) Internationale Anmeldenummer: PCT/EP1999/004192
(87) Internationale Veröffentlichungsnummer: WO 2000/016719

(56) Entgegenhaltungen:
- WO-A-97/12643
- WO-A-97/43961
- WO-A-98/19607
- WO-A-98/19629
- FR-A- 2 758 254
- US-A- 5 755 682

## Beschreibung

Die Erfindung betrifft eine Prothesen-Schlauchverbindung nach dem Oberbegriff des Anspruchs 1.

Bekannte Prothesen-Schlauchverbindungen bestehen im wesentlichen lediglich aus einem Prothesen-Schlauch aus primär dichtem, gebräuchlichem Prothesenmaterial. Zum Anschluß des Prothesenschlauches an die zu verbindenden Arterien, z.B. der Aorta abdominales, oder die Beckenarterie muß die Arterie an der Verbindungsstelle aufgeschnitten und durch eine Handnaht mit dem aus Kunststoff bestehenden Prothesenschlauch verbunden werden. Insbesondere bei minimal-invasiven Operationstechniken ist jedoch die Herstellung dieser Nähte nur mit großem Zeitaufwand und erheblichem Fehlerrisiko möglich. Man hat zwar schon versucht, die Nähte durch geeignete Apparate herzustellen, was jedoch sowohl vom erforderlichen Platzaufwand als auch hinsichtlich der Betriebssicherheit problematisch ist.

Aus der WO 97/43961 ist bereits eine Prothesen-Schlauchverbindung bekannt, bei der auf einem mit einem Längsschlitz versehenen, expandierbaren Metallgitter ein mit einem Rohrstutzen versehenes Schulterstück befestigt ist, wobei der Prothesenschlauch über einen aus beweglichen Teilen bestehenden Adapter mit dem Rohrstutzen verbindbar ist.

Aus der WO 98/19629 ist ein T-förmiger Flanschverbinder bekannt, der aus einem faltbaren Metallgitter und einer Ummantelung besteht. Der Querbalken des T-förmigen Verbinders wird in gefaltetem Zustand durch eine Öffnung in die Arterie eingebracht und dann durch ein Werkzeug expandiert. Danach erstreckt sich der Steg des T-förmigen Verbinders aus der Öffnung in der Arterie hinaus, so daß die Basis des T mit der Aorta verbunden werden kann.

Die FR-27 58 254 A offenbart einen am Ende mit einem rohrförmigen Abschnitt versehenen Prothesenschlauch, in dessen Endbereichen expandierbare Organe eingebracht oder von vornherein angeordnet sein können.

Die WO 98/ 19607 beschreibt die Verbindung einer Blutleitung mit einem dehnbaren Verbindungssegment aus Kunststoff durch eine Naht. Das Verbindungssegment weist einen nichtgefalteten mittleren Bereich auf, an dem die Naht vorgesehen ist. Aufblähbar sind nur die Endbereiche, die von der Verbindung mit dem Blutschlauch ausgenommen sind.

Das Ziel der Erfindung besteht darin, eine einfach aufgebaute und in Position bringbare künstliche Prothesen-Schlauchverbindung der eingangs genannten Gattung zu schaffen, mit der die Implantation der Gefäßprothese ohne die durch Herstellung einer Naht bedingte zeitliche Verzögerung möglich ist, wodurch die Operationszeiten erheblich verkürzt werden und die Gefahr eines Nahtbruches sowie einer Berstung der Verbindung zwischen Blutgefäß und Gefäßprothese gebannt werden soll.

Zur Lösung dieser Merkmale sind die Merkmale des kennzeichnenden Teils des Patentanspruchs 1 vorgesehen.

Der Erfindungsgedanke ist also darin zu sehen, daß eine z.B. für die Aufweitung von Arterien bekanntes rohrförmiges Metallgitter gegebenenfalls mit Innenverkleidung und/oder Ummantelung, welches auch als Stent bezeichnet wird, blutdicht mit einem T-artig abzweigenden, üblichen Prothesenschlauch unmittelbar verbunden wird. Das nur eine begrenzte Länge aufweisende gefaltete Metallgitter mit Innenverkleidung und/ oder Ummantelung kann bei auf das gefaltete Metallgitter abgeknickten Prothesenschlauch problemlos in eine mit der Öffnung versehene Arterie eingebracht und durch Klemmung, insbesondere mittels Selbst- oder Ballonexpansion fixiert werden, ohne daß es hierzu der Herstellung einer Naht bedarf. Das Metallgitter mit Innenverkleidung und/oder Ummantelung muß so lang sein, daß die beiden Endbereiche deutlich über das Ende des Arterienschnittes hinausragen und die Verklemmung innerhalb der Arterie gewährleisten können. Nach erfolgter Operation wächst dann der Schnitt so weit möglich wieder zu, wodurch die Halterung des Metallgitters innerhalb der Arterie weiter verbessert wird. Vorteilhafterweise wird auf besondere Bauteile wie Adapter oder Abzweigungen verzichtet, wodurch ein besonders einfacher Aufbau erzielt wird, indem lediglich das eine Ende des Prothesen-Schlauches unmittelbar an einem expandierbaren Stent angebracht, insbesondere angenäht wird. Ein besonderer Vorteil der unmittelbaren Verbindung von Metallgitter und Prothesenschlauch besteht darin, daß der Prothesenschlauch auf das gefaltete Metallgitter abgeknickt werden kann, wodurch die Einbringung des Metallgitters durch den Einschnitt in die Arterie problemlos erfolgen kann.

Außer einer schnellen und sicheren Anbringung der Prothese in einer Arterie wird auch das Infektionsrisiko durch die erfindungsgemäße Ausbildung herabgesetzt.

Durch die Maßnahmen der Ansprüche 2 und 3 wird gewährleistet, daß ein zunächst zusammengefaltetes oder zusammengedrücktes Metallgitter nach Einbringung in die Arterie so weit aufgedehnt werden kann, daß es sicher und fest innerhalb der Arterie sitzt.

Durch die Maßnahmen der Ansprüche 4 und 5 wird die Flexibilität des Schlauches bzw. der Ummantelung verbessert.

Die ovale Öffnung nach Anspruch 6 begünstigt den Blutfluß vom Inneren des Metallgitters in den Schlauch.

Die Verbindung des Schlauches mit dem Metallgitter bzw. der Innenverkleidung und/oder Ummantelung kann bevorzugt durch die im Anspruch 7 erwähnten Maßnahmen erfolgen.

Eine besonders stabile Anordnung wird durch die Ausführungsform nach Anspruch 8 erzielt, weil in diesem Fall die Metallgitter normalerweise vorhandenen Öffnungen auch zum Ausleiten des Blutes in den Schlauch herangezogen werden, ohne daß es der Anbringung einer zusätzlichen Öffnung im Metallgitter bedarf.

Vorteilhafte praktische Dimensionierungen der erfindungsgemäßen Prothesen-Schlauchverbindung entnimmt man den Patentansprüchen 9 bis 14.

Eine Variante könnte auch in einer gleichen Länge des stromaufwärtigen Teils, des stromabwärtigen Teils und der Anschlußstelle bestehen. Die Länge jedes dieser drei Elemente sollte etwa 1,7 cm betragen. Die optimalen Maße hängen vom Durchmesser des Metallgitters, dem Durchmesser der Metallgitteröffnung und dem Durchmesser des Prothesenschlauches ab.

Durch die Maßnahmen des Anspruchs 15 kann das Metallgitter mit Innenverkleidung und/oder Ummantelung einschließlich des Ballons im gefalteten Zustand problemlos durch die Öffnung in der Arterie in deren Inneres eingebracht und dann durch Aufblähen des Ballons auf den zum festen Sitz innerhalb der Arterie erforderlichen Durchmesser expandiert werden.

Anspruch 16 definiert eine für die gesteuerte Druckzuführung zum Ballon vorteilhafte Lösung.

Durch Ziehen der Reißleine, die durch die Öffnung nach außen geführt ist, kann gemäß Anspruch 17 das Metallgitter mit Innenverkleidung und/oder Ummantelung selbsttätig oder unterstützt durch einen eingebrachten Ballon in die endgültige Arbeitsposition gebracht werden.

Der Winkel, unter dem der dem Metallgitter zugewandte Endbereich des Schlauches relativ zum Metallgitter angeordnet ist, wird bevorzugt gemäß Anspruch 18 gewählt, weil aufgrund des schrägen Anschlusses des Endbereiches des Schlauches das Heranklappen desselben an das Metallgitter begünstigt und so dessen Einführung in die Arterie erleichtert wird.

Die Erfindung wird im folgenden beispielsweise anhand der Zeichnung beschrieben; in dieser zeigt:
- Figur 1: in einer schematischen Längsschnittansicht den einen Endbereich einer erfindungsgemäßen Prothesen-Schlauchverbindung nach Einbringung in eine Arterie,
- Figur 2: eine Draufsicht des Gegenstandes der Figur 1 nach Linie II-II in Figur 1,
- Figur 3: eine schematische Seitenansicht einer erfindungsgemäßen Prothesen-Schlauchverbindung im zusammengefalteten Zustand mit eingebrachtem Ballon und
- Figur 4: eine Ansicht analog Figur 3 nach dem Einbringen der Prothesen-Schlauchverbindung in eine Arterie und Aufblasen des Ballons.

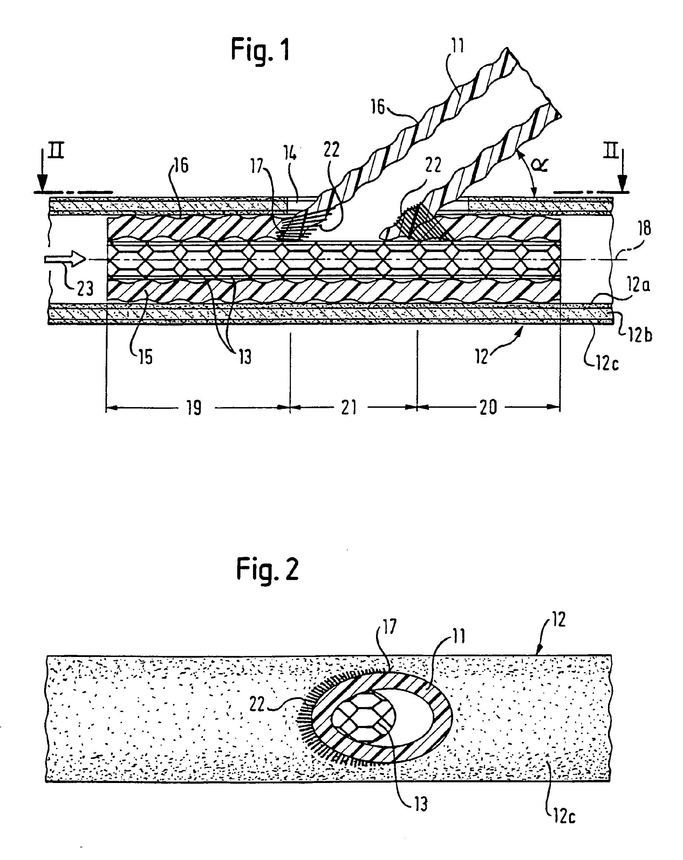

Nach Figur 1 ist ein rohrförmiges Metallgitter 13 mit einer Kunststoffummantelung 15 versehen. Derartig ummantelte Metallgitter (ummantelte Stents) sind aus der Aortenchirurgie bekannt, um beispielsweise die Hauptschlagader aufzuweiten bzw. aufgeweitete Arterien oder Aortenabschnitte im Durchmesser zu reduzieren bzw. geborstene Aortenabschnitte abzudichten.

Erfindungsgemäß ist die Ummantelung 15 an einer von beiden Enden entfernten Stelle auf ihrem Umfang mit einer in Richtung der Mittelachse 18 des Metallgitters 13, länglichen Öffnung 17 versehen, in die unter einem Winkel α das eine Ende eines Prothesenschlauches 11 eingesetzt ist, und zwar derart, daß das Material des Schlauches 11 bis zur Anlage am Metallgitter 13 kommt. In diesem Zustand wird das Ende des Schlauches 11 mit den die Öffnung 17 umgebenden Bereichen der Ummantelung 15 durch eine Naht 22 blutdicht verbunden. Auf diese Weise ist an dem mit der Ummantelung 15 versehenen Metallgitter 13 eine Anschlußstelle 21 für den Prothesenschlauch 11 gebildet, von der aus sich das Metallgitter 13 mit der Ummantelung 15 in beiden Längs-Richtungen erstreckt. Der sich entgegen der Blutstromrichtung 23 erstreckende Teil 19 ist dabei etwas länger als der sich in Blutstromrichtung 23 erstreckende Teil 20 des Metallgitters 13 mit der Ummantelung 15.

Wie aus Figur 2 ersichtlich ist, ist die Öffnung 17 aufgrund des schrägen Anschlusses des Endbereiches des Schlauches 11 an die Ummantelung 15 oval ausgebildet, wobei die längere Achse der Öffnung in Richtung der Mittelachse 18 des Metallgitters 13 weist.

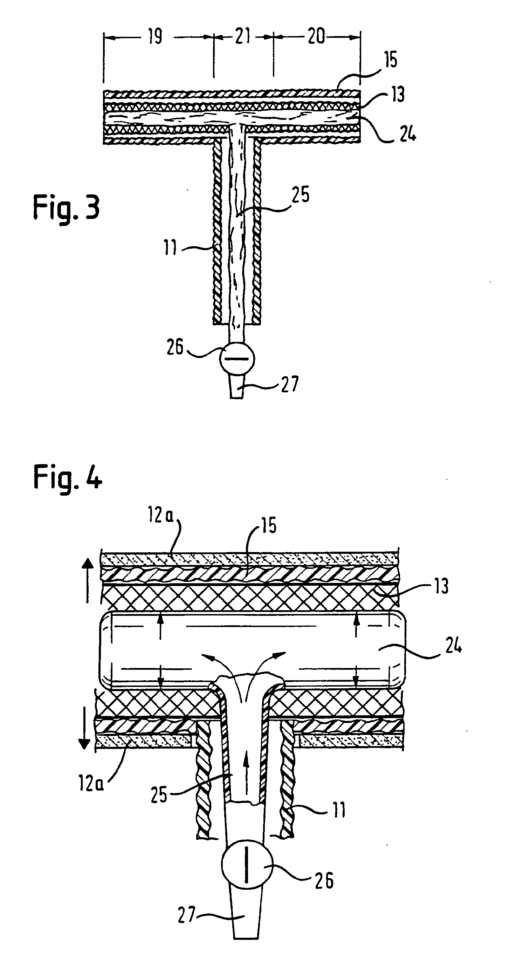

In den Figuren 1 und 2 ist die beschriebene Schlauchverbindung in einer Arterie 12 angeordnet dargestellt, welche aus drei Schichten besteht, und zwar der innenliegenden Intima 12a, der mittleren Media 12b und der außenliegenden Adventitia 12c. Eingebracht wird das Metallgitter 13 mit der Ummantelung 15 in die Arterie 12 dadurch, daß in dieser eine entsprechend der Öffnung 17 ovale Öffnung 14 vorgesehen wird. Anschließend wird dann zunächst der längere Teil 19 in gemäß Figur 3 gefalteter Form durch die Öffnung 14 in die Arterie 12 eingeschoben, und zwar so weit, daß anschließend auch der kürzere Teil 20 gefaltet in entgegengesetzter Richtung in die Arterie 12 eingebracht werden kann. Gemäß Figur 3 ist in das gefaltete Metallgitter ein sich in Richtung seiner Längsachse erstrekkender länglicher Ballon 24 in entspannter Form eingebracht, von dem im mittleren Bereich seitlich ein Zuleitungsschlauch 25 abzweigt, der durch den Schlauch 11 hindurchgeführt ist und in einem von Hand zu öffnenden bzw. zu schließenden Ventil 26 mündet, das einen Anschlußkonus 27 aufweist, welcher mit einer Druckluftquelle verbindbar ist.

Im Gegensatz zur Darstellung nach Figur 1 zweigt der Schlauch 11 in Figur 3 senkrecht vom Metallgitter 13 mit Ummantelung 15 ab.

Nachdem eine erfindungsgemäße Prothesen-Schlauchverbindung nach Figur 3 mit entspanntem Ballon 24 in eine Arterie 12 eingebracht worden ist, wird der Anschlußkonus 27 an die Druckluftquelle angeschlossen und das Ventil 26 geöffnet, worauf gemäß Figur 4 Druckluft in das Innere des Ballons strömen kann, so daß der Ballon sich aufbläht und eine Expansion des Metallgitters 13 mit der Ummantelung 15 so lange erfolgt, bis ein fester Sitz des Metallgitters 13 mit der Ummantelung 15 innerhalb der Arterie 12 gewährleistet ist, ohne daß es eines Vernähens bedarf.

Der Winkel α ist so gewählt, daß das in Richtung des Pfeiles 23 strömende Blut nicht unter einem Winkel von 90°, sondern vielmehr unter einem deutlich kleineren Winkel als 90°, beispielsweise 45° vom Inneren des Metallgitters 13 in den Schlauch 11 fließen kann. Ein Teil des Blutes kann jedoch durch das Metallgitter 13 hindurch am Schlauch 11 vorbei in den hinter dem Metallgitter befindlichen Teil der Arterie 12 fließen.

Am in der Zeichnung nicht dargestellten anderen Ende des Prothesen-Schlauches 11 kann ebenfalls ein Metallgitter 13 mit Ummantelung 15 ähnlich wie in Figur 1 dargestellt angeordnet sein, um auch das andere Ende des Schlauches 11 in ähnlicher Weise mit einer anderen Arterie bzw. einem anderen Arterienbereich verbinden zu können, ohne daß es zur Verbindung der Herstellung einer Naht bedarf.

Grundsätzlich kann statt der Ummantelung 15 oder zusätzlich zu dieser auch eine Innenverkleidung am Metallgitter 13 vorgesehen sein, die in der Zeichnung nicht gezeigt ist. In diesem Fall müßte die Innenverkleidung im Bereich der Anschlußstelle 21 mit einer geeigneten Durchlaßöffnung versehen sein. Gegebenenfalls kann auch das Metallgitter 13 im Bereich der Öffnung 17 eine durchgehende Öffnung aufweisen.

Sowohl die Ummantelung 15 als auch der Schlauch 16 sind mit einer Umfangsriffelung 16 versehen, wodurch die erforderliche Flexibilität des Schlauches 11 und der Kombination aus Metallgitter 13 und Ummantelung 15 erhöht wird.

Die Öffnung 17 soll in Richtung der Mittelachse 18 gesehen 0,8 bis 1,8 cm lang sein.

Die Einführung der in gefaltetem Zustand auf einem Ballonkatheter fixierten, erfindungsgemässen Prothesen-Schlauchverbindung in die Arterie oder Aorta kann auch über ein Einführungsbesteck, eine sogenannte Schleuse von maximal 12 mm Durchmesser erfolgen, was auch als Gefässpunktion bezeichnet wird. Das Einführungsbesteck ist konisch geformt und bewirkt eine Erweiterung der Arterie oder Schlagader am Punktionsort.

Auf diese Weise entfallen eine aufwendige Präparation und ein Abklemmen der Arterie oder Aorta sowie das chirurgische Öffnen des Gefässes.

## Patentansprüche

1. Prothesen-Schlauchverbindung zwischen zwei Arterien (12) bzw. Arterienbereichen innerhalb des menschlichen Körpers mit einem flexiblen, blutundurchlässigen Prothesenschlauch (11) geeigneter Länge, dessen Enden mit den zu verbindenden Arterien (12) in Strömungsverbindung stehen, wobei unter einem Winkel (α) zum Prothesenschlauch (11) ein eine vorzugsweise blutdichte Innenverkleidung und/oder Ummantelung (15) aufweisendes, gefaltetes rohrförmiges Metallgitter(Stent) (13) mit nach Entfaltung zumindest im Wesentlichen dem Durchmesser der Arterie (12) von solcher Länge vorgesehen ist, dass es in gefalteter Form durch eine in der Arterie (12) angebrachte Öffnung (14) in letztere so einführbar ist, dass es sich von der Öffnung (14) aus in beiden Richtungen der Arterie (12) erstreckt, und wobei die Innenverkleidung und/oder die Ummantelung (15) und ggf. auch das Metallgitter (13) eine mit dem Schlauchinnenraum kommunizierende Verbindungsöffnung (17) aufweisen und die Dimensionierung derart ist, dass das Metallgitter (13) mit Innenverkleidung und/oder Ummantelung (15) nach Entfaltung dicht in der Arterie (12) sitzt,
**dadurch gekennzeichnet, dass**
das Ende des Prothesenschlauches (11) unter dem Winkel (α) derart in die Verbindungsöffnung (17) eingesetzt ist, dass das Material des Prothesenschlauches (11) bis zur Anlage am Metallgitter (13) kommt und unmittelbar mit der Ummantelung (15) bzw. dem Metallgitter (13) und der Innenverkleidung verbunden ist.

2. Prothesen-Schlauchverbindung nach Anspruch 1,
**dadurch gekennzeichnet,**
**daß** das Metallgitter (13) ballonexpandierend ist.

3. Prothesen-Schlauchverbindung nach Anspruch 1,
**dadurch gekennzeichnet,**
**daß** das Metallgitter (13) selbstexpandierend ist.

4. Prothesen-Schlauchverbindung nach einem der Ansprüche 1 bis 3,
**dadurch gekennzeichnet,**
**daß** die Verkleidung und/oder Ummantelung (15) aus Dacron- oder Polytetrafluoräthylen besteht.

5. Prothesen-Schlauchverbindung nach einem der vorhergehenden Ansprüche,
**dadurch gekennzeichnet,**
**daß** der Schlauch (11) und/oder die Ummantelung (15) mit einer Umfangsriffelung (16) versehen sind.

6. Prothesen-Schlauchverbindung nach einem der Ansprüche 1 bis 5,
**dadurch gekennzeichnet,**
**daß** die Öffnung (17) rund oder oval ist, wobei die längere Achse der Öffnung (17) in Richtung der Mittelachse (18) des Metallgitters (13) verläuft.

7. Prothesen-Schlauchverbindung nach einem der vorhergehenden Ansprüche,
**dadurch gekennzeichnet,**
**daß** die Verbindung zwischen dem Schlauch (11) und dem Metallgitter (13) bzw. der Innenverkleidung und/oder der Ummantelung (15) durch Nähen, Weben, Schweißen oder Kleben erfolgt.

8. Prothesen-Schlauchverbindung nach einem der Ansprüche 1 bis 7,
**dadurch gekennzeichnet,**
**daß** das Metallgitter (13) im Bereich der Öffnung (17) durchgehend ist, d.h. keine über die Zwischenräume des Metallgitters (13) hinausgehenden Durchlaßöffnungen aufweist, oder eine entsprechend dem Durchmesser des Schlauches (11) dimensionierte Durchlassöffnung besitzt.

9. Prothesen-Schlauchverbindung nach einem der vorhergehenden Ansprüche,
**dadurch gekennzeichnet,**
**daß** das stromaufwärts von der Anschlußstelle (21) des Schlauches (11) gelegene Teil (19) des Metallgitters (13) und gegebenenfalls der Innenverkleidung und / oder Ummantelung (16) länger als das stromabwärts gelegene Teil (20) ist, wobei das stromaufwärts gelegene Teil (19) bevorzugt eine Länge von 1,5 bis 2 cm, insbesondere 1,7 cm und das stromabwärts gelegene Teil (20) vorzugsweise eine Länge von 1 bis 1,5 cm, insbesondere 1,3 cm aufweist.

10. Prothesen-Schlauchverbindung nach einem der vorhergehenden Ansprüche,
**dadurch gekennzeichnet,**
**daß** das stromaufwärts von der Anschlußstelle (21) des Schlauches (11) gelegene Teil (19) des Metallgitters (13) 10 bis 30 %, insbesondere etwa 20 % länger als die Anschlußstelle (21) des Schlauches (11) ist.

11. Prothesen-Schlauchverbindung nach einem der vorhergehenden Ansprüche,
**dadurch gekennzeichnet,**
**daß** das stromabwärts von der Anschlußstelle (21) des Schlauches (11) gelegene Teil (20) des Metallgitters (13) und gegebenenfalls der Innenverkleidung und/oder der Ummantelung (15) 5 bis 15 %, insbesondere etwa 20 % kürzer als die Anschlußstelle (21) des Schlauches (11) ist.

12. Prothesen-Schlauchverbindung nach einem der vorhergehenden Ansprüche,
**dadurch gekennzeichnet,**
**daß** der Schlauch (11) einen Durchmesser von 8 bis 18 mm hat und die Öffnung (17) in der Ummantelung (15) bzw. im Metallgitter und der Innenverkleidung entsprechend dimensioniert ist.

13. Prothesen-Schlauchverbindung nach einem der vorhergehenden Ansprüche,
**dadurch gekennzeichnet,**
**daß** die Länge des Metallgitters (13) und gegebenenfalls der Innenverkleidung und/oder der Ummantelung (15) 4,5 bis 5,5 cm beträgt.

14. Prothesen-Schlauchverbindung nach einem der vorhergehenden Ansprüche,
**dadurch gekennzeichnet,**
**daß** der Durchmesser des Metallgitters (13) bzw. der Ummantelung (15) 1,5 bis 2,5 cm beträgt.

15. Prothesen-Schlauchverbindung nach einem der vorhergehenden Ansprüche,
**dadurch gekennzeichnet,**
**daß** das Metallgitter (13) und gegebenenfalls die Innenverkleidung und/oder die Ummantelung (15) in gefaltetem Zustand mit einem entspannten Ballonkatheter in die Arterie (12) einbringbar und durch Aufblähung des Ballons (24) expandierbar sind.

16. Prothesen-Schlauchverbindung nach Anspruch 15,
**dadurch gekennzeichnet,**
**daß** von dem in das gefaltete Metallgitter (13) in entspannter Form einbringbaren, sich in Richtung seiner Längsachse erstreckenden länglichen Ballon (24) im mittleren Bereich seitlich ein Zuleitungsschlauch (25) abzweigt, der durch den Schlauch (11) hindurchgeführt ist und in einem von Hand zu öffnenden bzw. zu schließenden Ventil (26) mündet, das einen Anschlußkonus (27) aufweist, welcher mit einer Druckluftquelle verbindbar ist.

17. Prothesen-Schlauchverbindung nach Anspruch 15,
**dadurch gekennzeichnet,**
**daß** das zusammengefaltete Metallgitter (13) gegebenenfalls mit Innenverkleidung und/oder Ummantelung (15) durch eine Reißleine im gefalteten Zustand auf dem Katheter gehalten ist, wobei nach Ziehen an der Reißleine eine Selbst- oder Ballonexpansion des Metallgitters mit gegebenenfalls mit Innenverkleidung und/oder Ummantelung (15) erfolgt.

18. Prothesen-Schlauchverbindung nach einem der vorhergehenden Ansprüche,
**dadurch gekennzeichnet,**
**daß** der Winkel (α) 30° bis 60°, insbesondere etwa 45° beträgt.

## Claims

1. A prosthesis tube connection between two arteries (12), or arterial regions, inside the human body having a flexible, blood-impermeable prosthesis tube (11) of a suitable length whose ends are in flow communication with the arteries (12) to be connected, wherein a folded, tubular metal grid (stent) (13) is provided at an angle (α) to the prosthesis tube (11), said metal grid having an inner lining and/or jacket (15) which is preferably blood tight, having at least substantially the diameter of the artery (12) after unfolding and being of such a length that it can be introduced in folded form through an orifice (14) made in the artery into the former such that it extends starting from the orifice (14) in both directions of the artery (12), and wherein the inner lining and / or the jacket (15) and, optionally, also the metal grid (13) have a connecting orifice (17) communicating with the inner space of the tube and the dimensioning is such that the metal grid (13) having an inner lining and/ or jacket (15) sits tight in the artery (12) after unfolding,
**characterized in that**
the end of the prosthesis tube (11) is inserted into the connecting orifice (17) at the angle (α) such that the material of the prosthesis tube (11) comes into contact with the metal grid (13) and is connected directly to the jacket (15) or to the metal grid (13) and the inner lining.

2. A prosthesis tube connection in accordance with claim 1, **characterized in that** the metal grid (13) is balloon-expanding.

3. A prosthesis tube connection in accordance with claim 1, **characterized in that** the metal grid (13) is self-expanding.

4. A prosthesis tube connection in accordance with any one of the claims 1 to 3, **characterized in that** the lining and/or jacket (15) consists of Dacron or polytetra-fluoroethylene.

5. A prosthesis tube connection in accordance with any one of the preceding claims, **characterized in that** the tube (11) and/or the jacket (15) are provided with a peripheral corrugation (16).

6. A prosthesis tube connection in accordance with any one of the claims 1 to 5, **characterized in that** the orifice (17) is round or oval, with the longer axis of the orifice (17) extending in the direction of the central axis (18) of the metal grid (13).

7. A prosthesis tube connection in accordance with any one of the preceding claims, **characterized in that** the connection between the tube (11) and the metal grid (13) or the inner lining and/or the jacket (15) is carried out by suturing, weaving, welding or bonding.

8. A prosthesis tube connection in accordance with any one of the claims 1 to 7, **characterized in that** the metal grid (13) is continuous in the region of the orifice (17), i.e. has no passage openings going beyond the intermediate spaces of the metal grid (13), or has a passage opening dimensioned in accordance with the diameter of the tube (11).

9. A prosthesis tube connection in accordance with any one of the preceding claims, **characterized in that** the part (19) of the metal grid (13) and, optionally, of the inner lining and/or jacket (16), lying upstream of the connection point (21) of the tube (11) is longer than the part (20) lying downstream, with the part (19) lying upstream preferably having a length of 1.5 to 2 cm, in particular of 1.7 cm, and with the part (20) lying downstream preferably having a length of 1 to 1.5 cm, in particular of 1.3 cm.

10. A prosthesis tube connection in accordance with any one of the preceding claims, **characterized in that** the part (19) of the metal grid (13) lying upstream of the connection point (21) of the tube (11) is 10 to 30%, in particular approximately 20%, longer than the connection point (21) of the tube (11).

11. A prosthesis tube connection in accordance with any one of the preceding claims, **characterized in that** the part (20) of the metal grid (13) and, optionally, of the inner lining and / or the jacket (15) lying downstream of the connection point (21) of the tube (11) is 5 to 15%, in particular approximately 20%, shorter than the connection point (21) of the tube (11).

12. A prosthesis tube connection in accordance with any one of the preceding claims, **characterized in that** the tube (11) has a diameter of 8 to 18 mm and the orifice (17) in the jacket (15) or in the metal grid and the inner lining is correspondingly dimensioned.

13. A prosthesis tube connection in accordance with any one of the preceding claims, **characterized in that** the length of the metal grid (13) and, optionally, the inner lining and/or the jacket (15), amounts to 4.5 to 5.5 cm.

14. A prosthesis tube connection in accordance with any one of the preceding claims, **characterized in that** the diameter of the metal grid (13) or of the jacket (15) amounts to 1.5 to 2.5 cm.

15. A prosthesis tube connection in accordance with any one of the preceding claims, **characterized in that** the metal grid (13) and, optionally, the inner lining and/or the jacket (15), can be introduced into the artery (12) in a folded state using a collapsed balloon catheter and can be expanded by inflating the balloon (24).

16. A prosthesis tube connection in accordance with claim 15, **characterized in that** an infeed tube (25) branches off from the side in the central region of the elongate balloon (24) which can be introduced into the folded metal grid(13) in collapsed form and extends in the direction of its longitudinal axis, said infeed tube being guided through the tube (11) and opening into a valve (26) which can be opened and closed by hand and has a connection cone (27) which can be connected to a compressed air surce.

17. A prosthesis tube connection in accordance with claim 15, **characterized in that** the folded metal grid (13), optionally having an inner lining and/or jacket (15), is held on the catheter by a tear line in the folded state, with a self-expansion or balloon-expansion of the metal grid optionally having the inner lining and/or jacket (15) being carried out after pulling on the tear line.

18. A prosthesis tube connection in accordance with any one of the preceding claims, **characterized in that** the angle (α) amounts to 30° to 60°, in particular to approximately 45°.

## Revendications

1. Raccordement tubulaire prothétique entre deux artères (12) ou deux zones artérielles à l'intérieur du corps humain, comportant un tube prothétique (11) flexible imperméable au sang, de longueur appropriée, dont les extrémités sont en liaison d'écoulement avec les artères (12) à relier, dans lequel sous un angle (α) par rapport au tube prothétique (11) est prévue une grille métallique (stent) (13) tubulaire pliée présentant un revêtement intérieur et/ou une enveloppe (15) de préférence étanche au sang présentant au moins sensiblement le diamètre de l'artère (12) après son déploiement, d'une longueur telle que dans la forme pliée, ladite grille peut être introduite à travers une ouverture (14) ménagée dans l'artère (12) de telle manière qu'elle s'étend depuis l'ouverture (14) dans les deux directions de l'artère (12), et dans lequel le revêtement intérieur et/ou l'enveloppe (15), et le cas échéant aussi la grille métallique (13), présentent une ouverture de liaison (17) communiquant avec l'espace intérieur du tube, et les dimensions sont telles que la grille métallique (13) se trouve avec le revêtement intérieur et/ou l'enveloppe (15) hermétiquement dans l'artère (12) après le déploiement,
**caractérisé en ce que**
l'extrémité du tube prothétique (11) est insérée dans l'ouverture de liaison (17) sous un angle (α) de telle sorte que le matériau du tube prothétique (11) vient jusqu'en appui sur la grille métallique (13) et est relié directement à l'enveloppe (15) ou à la grille métallique (13) et au revêtement intérieur.

2. Raccordement tubulaire prothétique selon la revendication 1, **caractérisé en ce que** la grille métallique peut être expansée via un ballon.

3. Raccordement tubulaire prothétique selon la revendication 1, **caractérisé en ce que** la grille métallique (13) est autoexpansible.

4. Raccordement tubulaire prothétique selon l'une des revendications 1 à 3, **caractérisé en ce que** le revêtement et/ou l'enveloppe (15) est en Dacron ou en polytétrafluoroéthylène.

5. Raccordement tubulaire prothétique selon l'une des revendications précédentes, **caractérisé en ce que** le tube (11) et/ou l'enveloppe (15) sont pourvus d'un striage périphérique (16).

6. Raccordement tubulaire prothétique selon l'une des revendications 1 à 5, **caractérisé en ce que** l'ouverture (17) est ronde ou ovale, le grand axe de l'ouverture (17) s'étendant en direction de l'axe médian (18) de la grille métallique (13).

7. Raccordement tubulaire prothétique selon l'une des revendications précédentes, **caractérisé en ce que** la liaison entre le tube (11) et la grille métallique (13) ou le revêtement intérieur et/ou l'enveloppe (15) a lieu par couture, tissage, soudage ou collage.

8. Raccordement tubulaire prothétique selon l'une des revendications 1 à 7, **caractérisé en ce que** la grille métallique (13) est continue dans la région de l'ouverture (17), c'est-à-dire qu'elle ne présente aucune ouverture de passage en supplément aux interstices de la grille métallique (13), ou qu'elle présente une ouverture de passage correspondant au diamètre du tube (11).

9. Raccordement tubulaire prothétique selon l'une des revendications précédentes, **caractérisé en ce que** la partie (19), située en amont du point de raccordement (21) du tube, de la grille métallique (13), et le cas échéant du revêtement intérieur et/ou de l'enveloppe (16), est plus longue que la partie (20) située en aval, la partie (19) située en amont présentant de préférence une longueur de 1,5 à 2 cm, en particulier de 1,7 cm, et la partie (20) située en aval présentant de préférence une longueur de 1 à 1,5 cm, en particulier de 1,3 cm.

10. Raccordement tubulaire prothétique selon l'une des revendications précédentes, **caractérisé en ce que** la partie (19), située en amont du point de raccordement (21) du tube (11), de la grille métallique (13), est de 10 à 30 %, en particulier approximativement 20 % plus longue que le point de raccordement (21) du tube (11).

11. Raccordement tubulaire prothétique selon l'une des revendications précédentes, **caractérisé en ce que** la partie (20), située en aval du point de raccordement (21) du tube (11), de la grille métallique (13), et le cas échéant du revêtement intérieur et/ou de l'enveloppe (15), est de 5 à 15 %, en particulier approximativement 20 % plus courte que le point de raccordement (21) du tube (11).

12. Raccordement tubulaire prothétique selon l'une des revendications précédentes, **caractérisé en ce que** le tube (11) a un diamètre de 8 à 18 mm et l'ouverture (17) dans l'enveloppe (15) ou dans la grille métallique et le revêtement intérieur a des dimensions correspondantes.

13. Raccordement tubulaire prothétique selon l'une des revendications précédentes, **caractérisé en ce que** la longueur de la grille métallique (13), et le cas échéant du revêtement intérieur et/ou de l'enveloppe (15), est de 4,5 à 5,5 cm.

14. Raccordement tubulaire prothétique selon l'une des revendications précédentes, **caractérisé en ce que** le diamètre de la grille métallique (13) ou de l'enveloppe (15) est de 1,5 à 2,5 cm.

15. Raccordement tubulaire prothétique selon l'une des revendications précédentes, **caractérisé en ce que** la grille métallique (13), et le cas échéant le revêtement intérieur et/ou l'enveloppe (15), peut être introduite à l'état plié avec un cathéter à ballon détendu dans l'artère (12) et est expansible par gonflage du ballon (24).

16. Raccordement tubulaire prothétique selon la revendication 15, **caractérisé en ce qu'**à partir du ballon (24) allongé qui s'étend en direction de son axe longitudinal et qui, à l'état détendu, peut être introduit dans la grille métallique (13) pliée, un tube d'amenée (25) bifurque latéralement dans la région médiane, lequel est passé à travers le tube (11) et débouche dans une soupape (26) à ouvrir et à fermer à la main, laquelle présente un cône de raccordement (27) qui peut être relié à une source d'air comprimé.

17. Raccordement tubulaire prothétique selon la revendication 15, **caractérisé en ce que** la grille métallique (13) pliée est maintenue, le cas échéant avec le revêtement intérieur et/ou l'enveloppe (15), à l'état plié sur le cathéter par une cordelette à déchirer, et après avoir tiré sur la cordelette à déchirer, il se produit une expansion automatique ou via un ballon de la grille métallique le cas échéant avec le revêtement intérieur et/ou l'enveloppe (15).

18. Raccordement tubulaire prothétique selon l'une des revendications précédentes, **caractérisé en ce que** l'angle (α) est de 30° à 60°, en particulier approximativement de 45°.
